# FASCICULE DE BREVET EUROPEEN

(11) **EP 0 672 408 B1**
(45) Date de publication et mention de la délivrance du brevet: **20.06.2001**
(21) Numéro de dépôt: 95400249.9
(22) Date de dépôt: 06.02.1995
(51) Int. Cl.: A61K 7/135

(54) **Compositions cosmétiques pour la décoloration des cheveux, procédé de préparation et utilisation**
Kosmetische Zusammensetzungen zur Haarblondierung, Herstellungverfahren und Verwendung
Cosmetic compositions for hair bleaching, preparation process and use

(30) Priorité: 02.03.1994 FR 9402392
(43) Date de publication de la demande: 20.09.1995
(73) Titulaire: L'OREAL, 75008 Paris (FR)
(72) Inventeur: Tricaud, Caroline, F-95240 Cormeilles en Parisis (FR); Millequant, Jean, F-94100 Saint-Maur (FR); Sebag, Henri, F-75016 Paris (FR)
(74) Mandataire: Andral, Christophe André Louis

(56) Documents cités:
- EP-A- 0 574 696
- EP-A- 0 650 719
- EP-A- 0 663 205
- DE-A- 3 434 468
- FR-A- 2 276 809
- US-A- 4 327 751

## Description

La présente invention concerne des compositions cosmétiques décolorantes pour cheveux possédant des propriétés améliorées. Plus particulièrement encore, elle concerne des compositions de décoloration se présentant à l'état de poudres fines, anhydres, fluides, homogènes et non poussiérantes, qui se dispersent parfaitement dans l'eau oxygénée en générant, sans échauffement notable, des cataplasmes stables en viscosité, d'application aisée et qui présentent des propriétés cosmétiques améliorées.

Il est connu de décolorer les cheveux à l'aide de pâtes (ou cataplasmes) appliquées directement sur les cheveux et obtenues en mélangeant, au moment de l'emploi, une composition décolorante à base de composés peroxydés (agents oxydants) avec de l'eau, ou plus habituellement encore avec de l'eau oxygénée. La composition décolorante est, de façon connue, constituée d'un composé peroxydé (composant essentiel), généralement un persulfate ou un perborate de sodium, de potassium ou d'ammonium et parfois un sel d'acide percarboxylique ou un peroxyde, par exemple de baryum, de strontium, d'urée ou de mélamine. Le plus souvent, ces compositions contiennent en outre, de façon connue, des agents fortement alcalins tels que des métasilicates, des phosphates ou des carbonates alcalins ou alcalino-terreux (régulateurs de pH). Enfin, elles peuvent par ailleurs éventuellement contenir d'autres additifs ou adjuvants classiques dans le domaine : agents de controle du dégagement d'oxygène lors du mélange avec l'eau oxygénée, tels que carbonate de magnésium ou la magnésie; des épaississants, tels que des dérivés de cellulose (carboxyméthylcellulosse par exemple) ou l'amidon et ses dérivés, ou bien encore les gommes de guar, de xanthane et les alginates; des agents tensio-actifs, en particulier anioniques (alkylsulfates notamment); des colorants; des sequestrants; des parfums. De telles compositions décolorantes sont décrites, par exemple, dans "The Science of Hair Care" par C. ZVIAK, Marcel Decker Inc. 1986, pp 225-226.

Les compositions décolorantes pour cheveux les plus couramment utilisées à ce jour se présentent sous forme de poudres (mélanges) de faible granulométrie, c'est à dire avec des particules dont la taille est généralement inférieure au millimètre, de préférence inférieure à quelques centaines de microns, ce qui permet une dissolution et/ou une dispersion facile et rapide dans l'eau oxygénée.

Toutefois, de telles compositions pulvérulentes, compte tenu de l'état finement divisé dans lequel elles se trouvent, présentent plusieurs inconvénients : elles sont fortement volatiles et émettent ainsi, lors de leur manipulation, des poussières nocives contenant des composés peroxydés et fortement irritantes pour les poumons; ces poudres sont par ailleurs délicates non seulement à manipuler mais également à doser (problème de poussièrage et de coulabilité).

Pour tenter de résoudre les problèmes susmentionnés, on a décrit dans la demande de brevet EP-A- 0 560 088 une poudre décolorante pour cheveux totalement dépourvue de poussière (ou fines) et obtenue par ajout d'une huile ou d'une cire liquide au mélange de poudres initial (peroxyde solide + support solide à base des sels alcalins et des divers adjuvants décrits ci-avant).
Cependant, la poudre décolorante ci-dessus, bien qu'effectivement plus dense et moins poussiérante que les poudres décolorantes classiques, s'avère présenter d'autres désavantages, en particulier dus à la présence de l'huile ou de la cire, qui peuvent limiter fortement l'intérêt de son utilisation. Ainsi, avec cette poudre, on constate que les mélanges avec l'eau oxygénée sont longs à réaliser et donnent des cataplasmes brillants et d'aspect huileux; qu'une élimination poussée du produit des cheveux est longue et fastidieuse; que les shampooings utilisés pour faciliter cette élimination après les décolorations, ne moussent plus; et que finalement, après l'opération de décoloration, les cheveux gardent un toucher gras et lourd, désagréable.

Dans la demande de brevet EP-0650719 appartenant à l'état de la technique visé à l'Article 54 (3) CBE, on a décrit un procédé de fabrication de granulés pour décolorer les cheveux, caractérisé en ce qu'au cours de la granulation effectuée à température ambiante, on pulvérise un alcool sur un mélange en poudre, contenant au moins un persulfate minéral, des additifs habituels et un liant pouvant être un polyéthylèneglycol d'un poids moléculaire compris entre 100 et 600, dans la proportion de 1 à 20%, et que l'on sèche ensuite le granulé obtenu.

Par ailleurs, dans la demande de brevet DE-A-3 434 468, on a décrit un procédé d'obtention de composés d'addition du peroxyde d'hydrogène sous forme de poudres présentant, d'une part de bonnes propriétés d'écoulement et d'autre part, une bonne stabilité au stockage, par mélange à sec de ces composés sous forme de poudres, avec de 0,5 à 5,0% en poids d'un polyalkylèneglycol de poids moléculaire compris entre 200 et 4000 et comprenant 2 à 3 atomes de carbone dans le groupe alkyle. De telles poudres sont notamment utilisables pour la préparation de poudres décolorantes.

Cependant, avec de telles teneurs en polyalkylèneglycol, les cataplasmes obtenus par mélange de ces poudres avec l'eau oxygénée s'échauffent fortement, sont instables en viscosité au cours du temps, sont trop épais et difficiles à appliquer. En outre, les cheveux décolorés au moyen de ces cataplasmes sont rêches et difficiles à démêler, tant à l'état humide qu'à l'état sec.

Or, à la suite d'importantes recherches menées sur la question, il a maintenant été trouvé par la Demanderesse qu'il est possible d'obtenir sous la forme de poudres des compositions cosmétiques décolorantes pour cheveux ne présentant aucun des effets indésirables précités attachés aux compositions de l'art antérieur, en introduisant dans ces dernières un polymère spécifique, et dans la proportion de 10 à 27% en poids par rapport au poids total de la composition.

Cette découverte est la base de la présente invention.

Ainsi, selon la présente invention, il est maintenant proposé de nouvelles compositions cosmétiques anhydres pulvérulentes pour la décoloration des cheveux, à base de dérivés peroxydés comme agents oxydants, qui sont caractérisées par le fait qu'elles comprennent en outre à raison de 10 à 27% en poids par rapport à l'ensemble de la composition, d'au moins un polymère choisi au sein des polypropylèneglycols ou de leurs dérivés, ledit polymère étant à la fois anhydre et, à température ambiante, liquide d'une part et soluble dans l'eau d'autre part.

Le procédé de préparation des poudres décolorantes selon l'invention, lui-même constitutif d'un second objet de la présente invention, consiste à mélanger, en l'absence de solvant et à température ambiante, une poudre sèche décolorante à base de composés peroxydés solides, avec 10 à 27% en poids d'un polymère, qui est à la fois anhydre et, à température ambiante, liquide d'une part et soluble dans l'eau d'autre part, et choisi au sein des polyéthylèneglycols, des polypropylèneglycols et de leurs dérivés.
Selon l'invention, on dispose ainsi de compositions de décoloration se présentant sous la forme de poudres fines mais néanmoins denses et non volatiles (absence de poussiérage). Elles sont en outre anhydres et s'écoulent bien (faible mottage; facilité de dosage accrue). Par ailleurs, leur mélange avec l'eau oxygénée est rapide et aisé, et conduit à des cataplasmes s'échauffant peu et qui sont stables en viscosité au cours du temps; en outre ils sont très homogènes, moins épais et plus faciles à appliquer; les cheveux décolorés, humides ou secs, se démêlent mieux, sont moins rêches et mois crissants. D'autre part, ces cataplasmes sont onctueux, crémeux, sans grumeau, et d'aspect beaucoup plus agréable que ceux obtenus avec des poudres traitées par des huiles minérales, telles que des huiles de silicone ou des huiles de paraffine, ou des cires. En outre, l'application des cataplasmes est aisée et ceux-ci adhèrent bien aux cheveux et ne glissent pas. Enfin, l'élimination de ces derniers est nettement meilleure que dans le cas des poudres à l'huile ou à la cire; les shampooings moussent, les cheveux ne sont pas gras, restent brillants et gardent un toucher naturel.
Mais d'autres caractéristiques, aspects et avantages de l'invention apparaîtront encore plus clairement à la lecture de la description qui va suivre, ainsi que des divers exemples concrets, mais nullement limitatifs, destinés à l'illustrer.

Les polymères utilisés dans le cadre de la présente invention sont des produits déja bien connus en soi et aisément synthétisables par l'homme de l'art. Ils sont par ailleurs largement disponibles commercialement.
Comme indiqué précédemment, ils appartiennent à la famille générale des polyéthylèneglycols (PEG) ou des polypropylèneglycols (PPG), ou de leurs dérivés, c'est à dire des polymères dont la chaîne principale est constituée essentiellement d'une répétition de motifs dérivant de l'oxyde d'éthylène, respectivement de l'oxyde de propylène, ces polymères étant classiquement préparés selon une réaction de polyaddition, opérée de manière connue, en présence d'un initiateur de réaction qui peut être constitué par tout composé présentant un atome d'hydrogène labile (par exemple de l'eau, de l'éthylène glycol, du propylène glycol ou tout autre alcool ou polyol). A cet égard, par dérivés de polyéthylèneglycol ou de polypropylèneglycol, on entend plus particulièrement ici désigner et couvrir les produits de polyaddition qui sont obtenus en mettant en oeuvre des initiateurs autres que ceux qui sont habituellement utilisés à la préparation d'un polyéthylèneglycol ou d'un polypropylèneglycol vrai (i.e. eau, éthylèneglycol ou propylèneglycol).

Selon une caractéristique importante de la présente invention, on ne retient parmi les polymères ci-dessus que ceux qui sont (i) anhydres, (ii) liquides à température ambiante et (iii) solubles dans l'eau à température ambiante. Par température ambiante, on entend ici désigner une température qui peut être comprise entre 15°C et 30°C. Par ailleurs, par liquide, on entend plus particulièrement désigner un produit dont la viscosité est inférieure à 1000 mPa.s (centipoises), de préférence inférieure à 100 mPa.s (centipoises). Par anhydre, on entend que la teneur en eau du produit est inférieure à 1% en poids, de préférence inférieure à 0,5% en poids. Enfin, par soluble dans l'eau, on entend plus particulièrement viser un produit polymérique dont la solubilité dans l'eau est d'au moins 1 g/l, et de préférence d'au moins 10 g/l.

Selon la présente invention, il est bien entendu possible de mettre en oeuvre un ou plusieurs des polymères tels que ci-dessus définis.

La proportion en polymère(s) dans les compositions décolorantes selon l'invention est généralement comprise entre 10 et 27% en poids par rapport au poids total de ladite composition. De préférence, cette teneur est comprise entre 10 et 25% en poids, et encore plus préférentiellement, entre 10 et 20% en poids.

Les autres constituants essentiels (composés peroxydés à titre d'oxydants ) ou facultatifs (régulateurs de pH, épaississants, additifs cosmétiques) rentrant (ou pouvant rentrer) dans la composition des produits selon l'invention sont ceux que l'on rencontre habituellement pour des compositions décolorantes pour cheveux, notamment ceux indiqués dans la partie introductive de la présente description qui conviennent ici tout à fait.

A titre indicatif, les formulations décolorantes pour cheveux conformes à l'invention présentent généralement les compositions suivantes :
- agent(s) oxydant(s) (de préférence choisis parmi les persulfates d'alcalins) : de 20 à 60% en poids, de préférence de 30 à 50% en poids, par rapport à l'ensemble de la formulation ;
- polymère(s) : comme indiqué ci-avant ;
- régulateur(s) de pH (de préférence choisis parmi les métasilicates alcalins) : de 5 à 15% en poids, de préférence de 9 à 14% en poids, par rapport à l'ensemble de la formulation ;
- épaississant(s) : de 0,5 à 5% en poids, de préférence de 1 à 3% en poids, par rapport à l'ensemble de la composition ;
- adjuvant(s) cosmétique(s) éventuel(s) : qsp 100% en poids.

Comme indiqué précédemment, les compositions décolorantes selon l'invention se présentent dans un état solide pulvérulent. Les particules constituant cette poudre ont une taille généralement inférieure à 1000 µm (1 mm) mais surtout présentent une distribution granulométrique telle que le taux pondéral des particules qui ont une taille inférieure ou égale à 65 µm (taux de fines) est remarquablement faible. Ainsi, ce taux de fines est généralement inférieur à 1% en poids. Cette caractéristique fait que les poudres selon l'invention sont non poussiérantes. La taille maximale de particules indiquée ci-avant rend quant à elle les mélanges avec l'eau oxygénée beaucoup plus faciles à réaliser.

Selon un premier mode de réalisation du procédé de synthèse des compositions décolorantes selon l'invention, on introduit des poudres sèches décolorantes classiques à base de composés peroxydés (i.e des poudres que l'on désire en particulier rendre non volatiles) dans un mélangeur mécanique (mélangeur type LÖDIGE ou WINKWORTH notamment), ces poudres sont ensuite mises en agitation, puis on incorpore dans ces dernières le ou les polymères conformes à l'invention, et on mélange le tout, toujours par agitation mécanique, pour homogénéisation, et enfin on récupère le produit résultant qui constitue la composition selon l'invention.

Selon un autre mode de réalisation, on pulvérise simplement (par exemple par atomisation) le ou les polymères liquides conformes à l'invention sur les poudres décolorantes initiales maintenues en agitation (agitation mécanique ou par lit fluidisé par exemple), puis on récupère le produit résultant qui constitue la composition selon l'invention.

Les deux modes de réalisation ci-dessus se font à température ambiante et sans mettre en oeuvre de tiers solvants, tels que de l'eau ou des solvants organiques (mélange à sec), même au niveau de l'étape de l'introduction du polymère. Les proportions poudre initiale/polymère(s) sont choisies de la même manière qu'indiquée ci-avant pour les compositions finales désirées. Dans les deux cas, les temps de mélange permettent de contrôler les granulométries finales.

Les compositions décolorantes pulvérulentes conformes à l'invention peuvent ensuite être mises en oeuvre sur les cheveux de manière classique et connue en soi dans le domaine de la décoloration capillaire. Ainsi, par exemple, on mélange les compositions décolorantes sous forme de poudres avec une solution d'eau oxygénée à 6-12 % en volume, de préférence à 9 % environ en volume, et ceci dans un rapport généralement de l'ordre de 1 : 1, puis on mélange jusqu'à l'obtention d'une pâte homogène que l'on applique ensuite sur les cheveux et que l'on laisse pauser pendant une durée comprise entre 25 et 45 minutes. La composition est ensuite éliminée des cheveux (rinçage à l'eau et/ou shampooings).

Des exemples concrets illustrant l'invention vont maintenant être donnés.

### EXEMPLE 1

On a préparé une composition décolorante (C1) qui présentait la composition suivante (% en poids) :
- Persulfate de potassium 40%
- Persulfate d'ammonium 15%
- Métasilicate de sodium 12%
- Chlorure d'ammonium 5%
- Séquestrant 1%
- Silice 7%
- polymère ^{**(1)**} 20%

^{**(1)**}: polyéthylèneglycol à 80 moles d'OE, de poids moléculaire moyen 400, vendu sous le nom commercial de BREOX PEG 400 par la Société BP.

Le mode opératoire était le suivant : on a introduit dans un mélangeur de type LÖDIGE les différents composés solides constituant la poudre décolorante initiale (i.e ne contenant pas le polymère), on les a mélangés à sec pendant 20 minutes dans le mélangeur, puis on a introduit dans ce dernier le polymère servant d'agent d'enrobage, et on a mélangé le tout (toujours à sec) pour homogénéisation pendant 20 minutes.

La granulométrie de la poudre décolorante finale obtenue (déterminée par tamisage) est indiquée dans le tableau donné ci-après (C1). Ce tableau indique le % en poids des particules présentes dans la composition dont la taille est comprise dans un intervalle donné de taille.

A titre comparatif, on a également indiqué dans ce tableau la granulométrie de la poudre décolorante avant l'introduction du polymère (C0).

La composition C1, mélangée poids pour poids avec de l'eau oxygénée à 80 volumes, ne s' échauffe en 10 minutes que de 6°C alors qu' un mélange similaire d'une composition C'1 (laquelle ne contient que 5% de polymère 1 au lieu de 20%, toutes choses égales par ailleurs) s' échaufffe de 18°C.

En outre, la composition C1, mélangée poids pour poids avec de l'eau oxygénée à 30 volumes, conduit à un cataplasme de viscosité initiale égale à 174 mPa.s (cp) et de viscosité égale à 194 mPa.s (cp) après 30 minutes de temps de pause. Dans ces conditions, la composition comparative C'1 conduit à un cataplasme dont la viscosité initiale de 2870 mPa.s (cp) chute à 1880 mPa.s (cp).

Les cheveux décolorés avec la composition C1 sont doux et faciles à démêler. Ceux décolorés avec la composition C'1 sont plus rêches, et difficiles à démêler.

### EXEMPLE 2

En reprenant le mode opératoire décrit à l'exemple 1, on a préparé une deuxième composition conforme à l'invention (C2) semblable à celle de l'exemple 1 à ces deux seules différences près que le polymère 1 a été remplacé ici par un polypropylèneglycol présentant un poids moléculaire moyen de 260 vendu sous le nom commercial de PPG PM 260 par la Société ALDRICH et que celui-ci a été utilisé à raison de 18% en poids.

La granulométrie de la poudre décolorante finale obtenue (déterminée par tamisage) est indiquée dans le tableau donné ci-après (C2).

### EXEMPLE 3

En reprenant le mode opératoire décrit à l'exemple 1, on a préparé une troisième composition conforme à l'invention (C3) semblable à celle de l'exemple 1 à ces deux seules différences près que le polymère 1 a été remplacé ici par un polypropylèneglycol présentant un poids moléculaire moyen de 725 vendu sous le nom commercial de PPG PM 725 par la Société ALDRICH et que celui-ci a été utilisé à raison de 18% en poids.

La granulométrie de la poudre décolorante finale obtenue (déterminée par tamisage) est indiquée dans le tableau donné ci-après (C3).

### EXEMPLE 4

En reprenant le mode opératoire de l'exemple 1, on a préparé une quatrième composition conforme à l'invention (C4) présentant cette fois la composition pondérale suivante :
- Persulfate de potassium 25%
- Persulfate d'ammonium 25%
- Métasilicate de sodium 10%
- Chlorure d'ammonium 5%
- Séquestrant 2%
- Silice 13%
- Polymère 4^{**(2)**} 20%

^{**(2)**} : PPG-10 Butanediol (CTFA) vendu sous le nom commercial de MACOL 57 par la Société PPG Industries.

La granulométrie de la poudre décolorante finale obtenue (déterminée par tamisage) est indiquée dans le tableau donné ci-après (C4).

La granulométrie du mélange de poudres avant l'introduction du polymère était identique à celle de la composition C0 de l'exemple 1.

**TABLEAU**

| Taille Φ (µm) | C 0 | C 1 | C 2 | C 3 | C 4 |
|---|---|---|---|---|---|
| Φ>710 | 2,2 | 47,50 | 31,04 | 27,70 | 32,00 |
| 355< Φ <710 | 7,8 | 19,34 | 22,48 | 30,94 | 50,60 |
| 100< Φ <355 | 28,46 | 17,94 | 20,74 | 17,92 | 13,11 |
| 60< Φ <100 | 52,86 | 13,72 | 25,40 | 13,00 | 3,26 |
| 0< Φ <60 | 7,74 | 0,40 | 0,24 | 0,16 | 0,00 |

Les quatre compositions 1, 2, 3 et 4 étaient dépourvues de toute poussière.

## Revendications

1. Compositions cosmétiques anhydres pulvérulentes pour la décoloration des cheveux, à base de composés peroxydés comme agents oxydants, caractérisées par le fait qu'elles comprennent, à raison de 10 à 27% en poids, d'au moins un polymère choisi au sein des polypropylèneglycols ou de leurs dérivés, qui est à la fois anhydre et, à température ambiante, liquide d'une part et soluble dans l'eau d'autre part.

2. Compositions selon la revendication 1, caractérisées en ce que la teneur en polymère(s) est comprise entre 10 et 25% en poids par rapport au poids total de la composition.

3. Compositions selon la revendication 2, caractérisées en ce que ladite teneur est comprise entre 10 et 20% en poids.

4. Compositions selon l'une quelconque des revendications précédentes, caractérisées en ce que la teneur en agents oxydants de type composés péroxydés est comprise entre 20 et 60% en poids, de préférence de 30 à 50% en poids, par rapport à l'ensemble de la formulation.

5. Compositions selon la revendication 4, caractérisées en ce que les composés péroxydés sont choisis parmi des persulfates d'alcalins.

6. Compositions selon l'une quelconque des revendications précédentes, caractérisées en ce que le taux pondéral des particules les constituant qui ont une taille inférieure ou égale à 65 µm est inférieur à 1% en poids.

7. Compositions selon l'une quelconque des revendications précédentes, caractérisées par le fait que la taille des particules les constituant ont une taille inférieure à 1000 µm (1 mm).

8. Procédé de préparation d'une composition anhydre pulvérulente pour la décoloration des cheveux, caractérisé en ce qu'il consiste à mélanger, en l'absence de solvant et à température ambiante, une poudre sèche décolorante à base de composés peroxydés solides, avec 10 à 27% en poids d'un polymère, qui est à la fois anhydre et, à température ambiante, liquide d'une part et soluble dans l'eau d'autre part, et choisi au sein des polypropylèneglycols et de leurs dérivés.

9. Procédé de préparation d'une composition anhydre pulvérulente pour la décoloration des cheveux, caractérisé en ce qu'il consiste à mélanger, en l'absence de solvant et à température ambiante, une poudre sèche décolorante à base de composés peroxydés solides, avec 10 à 27% en poids d'un polymère, qui est à la fois anhydre et, à température ambiante, liquide d'une part et soluble dans l'eau d'autre part, et choisi au sein des polyéthylèneglycols, et de leurs dérivés.

10. Utilisation d'une composition telle que définie à l'une quelconque des revendications 1 à 7 ou obtenue selon le procédé de la revendication 8 pour la décoloration des cheveux.

11. Procédé de décoloration des cheveux, caractérisé par le fait qu'il consiste à mélanger une composition telle que définie à l'une quelconque des revendications 1 à 7 ou obtenue selon le procédé de la revendication 8 avec de l'eau oxygénée, à appliquer le mélange résultant sur des cheveux, à laisser pauser, et enfin à éliminer la composition des cheveux.

## Claims

1. Pulverulent anhydrous cosmetic compositions for bleaching hair, based on peroxide compounds as oxidizing agents, characterized by the fact that they comprise, at a rate of 10 to 27% by weight, at least one polymer chosen from polypropylene glycols or derivatives thereof, which is both anhydrous and, at room temperature, is firstly liquid and secondly watersoluble.

2. Compositions according to Claim 1, characterized in that the content of polymer(s) is between 10 and 25% by weight relative to the total weight of the composition.

3. Compositions according to Claim 2, characterized in that the said content is between 10 and 20% by weight.

4. Compositions according to any one of the preceding claims, characterized in that the content of oxidizing agents of the peroxide compound type is between 20 and 60% by weight, preferably 30 to 50% by weight, relative to the whole formulation.

5. Compositions according to Claim 4, characterized in that the peroxide compounds are chosen from alkali metal persulphates.

6. Compositions according to any one of the preceding claims, characterized in that the quantity by weight of the particles constituting them which have a size less than or equal to 65 µm is less than 1% by weight.

7. Compositions according to any one of the preceding claims, characterized by the fact that the size of the particles constituting them have a size less than 1000 µm (1 mm).

8. Process for preparing a pulverulent anhydrous composition for bleaching the hair, characterized in that it consists in mixing, in the absence of solvent and at room temperature, a dry bleaching powder based on solid peroxide compounds, with 10 to 27% by weight of a polymer, which is both anhydrous and, at room temperature, firstly liquid and secondly water-soluble, and chosen from polypropylene glycols and derivatives thereof.

9. process for preparing a pulverulent anhydrous composition for bleaching the hair, characterized in that it consists in mixing, in the absence of solvent and at room temperature, a dry bleaching powder based on solid peroxide compounds, with 10 to 27% by weight of a polymer, which is both anhyrous and, at room temperature, firstly liquid and secondly water-soluble, and chosen from polyethylene glycols and derivatives thereof.

10. Use of a composition as defined in any one of Claims 1 to 7 or which is obtained according to the process of Claim 8 for bleaching the hair.

11. Process for bleaching the hair, characterized by the fact that it consists in mixing a composition as defined in any one of Claims 1 to 7 or which is obtained according to the process of Claim 8, with hydrogen peroxide, in applying the resulting mixture to the hair, in allowing to act, and finally in removing the composition from the hair.

## Patentansprüche

1. Wasserfreie kosmetische Zusammensetzungen in Pulverform zum Blondieren der Haare auf der Basis von Peroxidverbindungen als Oxidationsmittel, dadurch gekennzeichnet, daß sie in einem Mengenanteil von 10 bis 27 Gew.-% mindestens ein Polymer enthalten, das unter den Polypropylenglykolen oder deren Derivaten ausgewählt ist, das wasserfrei ist und das bei Raumtemperatur flüssig und in Wasser löslich ist.

2. Zusammensetzungen nach Anspruch 1, dadurch gekennzeichnet, daß der Mengenanteil des Polymers oder der Polymere im Bereich von 10 bis 25 Gew.-%, bezogen auf das Gesamtgewicht der Zusammensetzung, liegt.

3. Zusammensetzungen nach Anspruch 2, dadurch gekennzeichnet, daß der Mengenanteil im Bereich von 10 bis 20 Gew.-% liegt.

4. Zusammensetzungen nach einem der vorhergehenden Ansprüche, dadurch gekennzeichnet, daß der Mengenanteil der Oxidationsmittel vom Peroxidtyp im Bereich von 20 bis 60 Gew.-% und vorzugsweise 30 bis 50 Gew.-%, bezogen auf die gesamte Formulierung, liegt.

5. Zusammensetzungen nach Anspruch 4, dadurch gekennzeichnet, daß die Peroxide unter den Alkalipersulfaten ausgewählt sind.

6. Zusammensetzungen nach einem der vorhergehenden Ansprüche, dadurch gekennzeichnet, daß der Gewichtsanteil der Partikel, die die Zusammensetzungen bilden und die eine Größe von 65 µm oder darunter aufweisen, unter 1 Gew.-% liegt.

7. Zusammensetzungen nach einem der vorhergehenden Ansprüche, dadurch gekennzeichnet, daß die Größe der Partikel, die die Zusammensetzung bilden, unter 1000 µm (1 mm) liegt.

8. Verfahren zur Herstellung einer wasserfreien Zusammensetzung in Pulverform zum Blondieren der Haare, dadurch gekennzeichnet, daß es darin besteht, ein trockenes Blondierpulver auf der Basis von festen Peroxiden ohne Lösungsmittel bei Raumtemperatur mit 10 bis 27 Gew.-% eines Polymers zu vermischen, das wasserfrei, bei Raumtemperatur flüssig und in Wasser löslich und unter den Polypropylenglykolen und deren Derivaten ausgewählt ist.

9. Verfahren zur Herstellung einer wasserfreien Zusammensetzung in Pulverform zum Blondieren der Haare, dadurch gekennzeichnet, daß es darin besteht, ein trockenes Blondierpulver auf der Basis von festen Peroxiden ohne Lösungsmittel bei Raumtemperatur mit 10 bis 27 Gew.-% eines Polymers zu vermischen, das wasserfrei, bei Raumtemperatur flüssig und in Wasser löslich und unter den Polyethylenglykolen und deren Derivaten ausgewählt ist.

10. Verwendung einer Zusammensetzung nach einem der Ansprüche 1 bis 7 oder einer nach dem Verfahren des Anspruchs 8 hergestellten Zusammensetzung zum Blondieren der Haare.

11. Verfahren zum Blondieren der Haare, dadurch gekennzeichnet, daß es darin besteht, eine Zusammensetzung nach einem der Ansprüche 1 bis 7 oder eine nach dem Verfahren des Anspruchs 8 hergestellte Zusammensetzung mit einer Wasserstoffperoxidlösung zu vermischen, das resultierende Gemisch auf das Haar aufzubringen und einwirken zu lassen und schließlich die Zusammensetzung aus dem Haar zu entfernen.
